Europäisches Patentamt

(19) **European Patent Office**

**Office européen des brevets**

(11) Publication number: **0 021 847**
**B1**

(12) **EUROPEAN PATENT SPECIFICATION**

(45) Date of publication of patent specification: **20.07.83**

(51) Int. Cl.³: **A 61 K 9/08, A 61 K 31/63**

(21) Application number: **80302190.6**

(22) Date of filing: **30.06.80**

(54) Long acting sulfonamide injectable compositions.

(30) Priority: **02.07.79 US 54313**

(43) Date of publication of application:
**07.01.81 Bulletin 81/1**

(45) Publication of the grant of the patent:
**20.07.83 Bulletin 83/29**

(84) Designated Contracting States:
**AT BE CH DE FR GB IT LI LU NL SE**

(56) References cited:
**DE - A - 2 631 779**
**FR - A - 2 340 100**
**GB - A - 1 538 903**
**GB - A - 2 000 970**
**US - A - 3 985 876**
**US - A - 4 018 889**

**MERCK INDEX, 9th Edition, 1976, Merck & Co.,
page 1153, RAHWAY (US)**

The file contains technical information
submitted after the application was filed and not
included in this specification

(73) Proprietor: PFIZER INC.
235 East 42nd Street
New York, N.Y. 10017 (US)

(72) Inventor: Armstrong, William Wellesley
Calf Farm Road
Mill Neck, Nassau, N.Y. (US)
Inventor: Desai, Saurabhkumar Jayavantrai Sheela
Apartments
Flat No. 30 Mahalaxmi Warden Road
Bombay 26 (IN)

(74) Representative: Wood, David John et al,
Pfizer Limited Ramsgate Road
Sandwich Kent CT13 9NJ (GB)

Long acting sulfonamide injectable compositions

This invention relates to sulfonamide solutions suitable for pharmaceutical use. More particularly, it relates to long acting aqueous injectable solutions of sulfadimethoxime in 2-pyrrolidone.

Parenteral solutions of sulfamethoxazole and trimethoprim containing the following water-miscible solvents are disclosed in U.S. Patent No. 3,551,564: polyethyleneglycol ethers of tetrahydrofurfuryl alcohol, dimethylformamide, dimethylsulfoxide, dimethylacetamide, diethylacetamide, 1,2-propyleneglycol, di(1,2-propyleneglycol) 1,3-butylene glycol, glycerol formal, diethylene glycol and polyethylene glycol containing 2—15 ethylene oxide groups.

U.S. Patent No. 3,985,876 discloses solutions of trimethoprim with various sulfonamides, including sulfamethoxazole and sulfadoxine in a mixture of 10—60% water and 30—90% organic solvents such as dimethyl and diethylacetamide, dimethyl formamide, dimethylsulfoxide, glycerol formal and various glycols.

U.S. Patent No. 4,018,889 discloses oxytetracycline solutions containing from about 1 to 40% oxytetracycline in an aqueous vehicle containing from about 10 to 50% by weight of 2-pyrrolidone, about 0.8 to 1.3 molar proportions of a pharmaceutically acceptable magnesium compound soluble in the said solution, said solution having a pH value in the range of from about 7.5 to 9.5.

French patent No. 2340100 discloses stable injectable sulphanomide preparations at pH 5.5—7.5 comprising a solution of the diethanolamine salt of a sulphonamide (e.g. sulfafurazole) and a salt of a sulphonamide protentiator (e.g. diavendine or trimethoprim lactate) in a mixture of water, propylene glycol and N-methylpyrrolidone in stated proportions.

German Offenlegungsschrift No. 2631779 discloses clear aqueous sulfonamide-trimethoprim solutions, characterised by containing a water-soluble sulphonamide salt, trimethoprim and polyvinylpyrrolidone with a K-value of 10—18 in water.

British Patent No. 1,538,903 discloses a composition comprising a physiologically active agent (e.g. a sulphonamide) or cosmetic agent and a topical pharmaceutical carrier composition containing at least 10% by weight of the carrier composition of a vehicle system comprising a mixture of 2-pyrrolidone and N-methyl-2-pyrrolidone in a weight ratio of between 1:4 and 4:1.

This invention discloses a long acting liquid sulfonamide injectable composition comprising (a) water (b) from 5 to 25% w/v based on the total volume of the solution of sulfadimethoxine and (c) 40 to 65% w/v based on the total volume of the solution of a co-solvent, which is 2-pyrrolidone, the composition having a pH of from 5 to 9.5.

The preferred concentration for sulfadimethoxine is 10 to 20% w/v.

Sulfadimethoxine is also known as N¹-(2,6-dimethoxy-4-pyrimidinyl)sulfanilamide. The preferred concentration for 2-pyrrolidone is from 50 to 65% w/v.

The pH value of these compositions is adjusted if necessary to pH 5 to 9.5. The pH can be adjusted by means of a base that is pharmaceutically acceptable, such as monoethanolamine.

As an optional ingredient polyvinylpyrrolidone, having a molecular weight of between 5,000 and 100,000 (K-12 to 30), may also be present in these compositions in a concentration of from 1 to 5% w/v. The polyvinylpyrrolidone most preferred for this invention is one having an average molecular weight of about 10,000—17,000 (where K-value=17). It is present in part as a co-solubilizer and may improve tissue toleration.

As optional co-solvents ingredients such as propylene glycol, polyethylene glycols, benzyl alcohol, dimethylacetamide, ethyl lactate and glycerol formal may be present at concentrations of from 0.5 to 30% w/v.

The stability of these solutions for therapeutic administration is still further enhanced by use of an antioxidant such as monothioglycerol at levels of from 0.1 to 1% w/v.

The primary application of these sulfonamide compositions is as a parenteral composition.

The sulfonamide antibiotic compositions of this invention are easy to syringe over a wide temperature range and are satisfactory from a physical and chemical stability standpoint.

These solutions produce prolonged efficacious blood levels and are well tolerated in contrast to the short term blood levels and tissue damage routine experienced with presently available highly alkaline aqueous sulfonamide solutions.

The compositions of this invention are preferably prepared by mixing the co-solvent with water and other co-solvents, if present. The sulfonamide is then added and stirred until a clear solution results. The pH is then adjusted to the desired range. If polyvinylpyrrolidone is to be included, it is added to the water at the time of mixing the co-solvent.

The present invention is illustrated by the following Examples:

Example 1

|  | g/100 ml |
| --- | --- |
| Sulfadimethoxine | 5.00 |
| Pyrrolidone | 50.00 |
| Dimethylacetamide | 10.00 |

Polyvinylpyrrolidone
| | |
|---|---|
| (K=17) | 5.00 |
| Ethyl lactate | 10.00 |
| Benzyl alcohol | 0.90 |
| Monothioglycerol | 0.20 |
| Water q.s. to | 100 ml |

The 2-pyrrolidone, dimethylacetamide, polyvinylpyrrolidone, ethyl lactate, benzyl alcohol and monothioglycerol were mixed and dissolved, successively, in water. The sulfadimethoxine was added and stirred until dissolved. The solution was then brought up to volume with water. The pH of the solution was 6.5 and the viscosity was 21 cts at 25°C.

Example 2

The following solution was prepared using the procedure of Example 1.

| | g/100 ml |
|---|---|
| Sulfadimethoxine | 10.00 |
| 2-Pyrrolidone | 65.00 |
| Polyvinylpyrrolidone | |
| (K=17) | 5.00 |
| Ethyl lactate | 15.00 |
| Benzyl alcohol | 0.90 |
| Monothioglycerol | 0.20 |
| Water q.s. to | 100 ml |

The pH of the solution was 6.8 and the viscosity was 36 cts at 25°C.

Example 3

The following solution was prepared using the procedure of Example 1.

| | g/100 ml |
|---|---|
| Sulfadimethoxine | 20.00 |
| 2-Pyrrolidone | 55.00 |
| Dimethylacetamide | 10.00 |
| Polyvinylpyrrolidone | |
| (K—17) | 5.00 |
| Ethyl lactate | 7.00 |
| Benzyl alcohol | 0.90 |
| Monothioglycerol | 0.20 |
| Water q.s. to | 100 ml |

The pH of the solution was 6.7 and the viscosity was 28 cts at 25°C.

Raising the pH of the above solution with monoethanolamine to 9.5 produced a similar solution with a viscosity of 61 cts. Lowering the pH with hydrochloric acid to 5.0 produced a similar solution with pH 7.0 and a viscosity of 35 cts.

The substitution of glycerol formal for ethyl lactate produced a similar solution with pH 7.3 and a viscosity of 44 cts.

Example 4

| | g/100 ml |
|---|---|
| Sulfadimethoxine | 25.00 |
| 2-Pyrrolidone | 60.00 |
| Dimethylacetamide | 10.00 |

| | |
|---|---|
| Ethyl lactate | 7.00 |
| Benzyl alcohol | 0.90 |
| Monothioglycerol | 0.20 |
| Water q.s. to | 100 ml |

The solution was prepared by dissolving all the ingredients in water, with the sulfadimethoxine being added last. The pH of the solution was 6.8.

Example 5

| | g/100 ml |
|---|---|
| Sulfadimethoxine | 20.00 |
| 2-Pyrrolidone | 63.50 |
| Ethyl lactate | 15.00 |
| Polyvinylpyrrolidone | |
| (K=17) | 4.20 |
| Benzyl alcohol | 0.90 |
| Monothioglycerol | 0.20 |
| Water q.s. to | 100 ml |

The solution was prepared using the procedure of Example 1. The pH of the solution was 6.7 and the viscosity was 35 at 25°C.

Example 6

Formulations containing 200 mg/ml of sulfadimethoxine in aqueous 2-pyrrolidone were administered by intramuscular injection to swine at a dose of 55 mg/kg. The plasma levels after injection are give below. The control solution contained 200 mg/ml sulfadimethoxine as the sodium salt in water at pH 10.4.

| Time post dose (hours) | Example 3 | Example 5 | Control |
|---|---|---|---|
| 4 | 47 | 55 | 94 |
| 7 | 49 | 54 | 103 |
| 24 | 37 | 46 | 24 |
| 48 | 22 | 26 | 6 |
| 72 | 15 | 15 | 3 |
| 96 | 8 | 10 | — |
| 120 | 6 | 8 | — |

As can be seen, by using 2-pyrrolidone as a co-solvent the plasma levels are prolonged when compared to results obtained with the aqueous sodium salt solution.

**Claims for the contracting states: BE, CH, DE, FR, GB, IT, LI, LU, NL, SE**

1. A long acting liquid sulfonamide injectable composition comprising (a) water, (b) from 5 to 25% w/v based on the total volume of the solution of sulfadimethoxine and (c) 40 to 65% w/v based on the total volume of the solution of a co-solvent which is 2-pyrrolidone, the composition having a pH of from 5 to 9.5.

2. A composition as claimed in claim 1 which further comprises from 1 to 5% w/v based on the total volume of the solution of polyvinylpyrrolidone having a m.w. of 5000—100,000.

3. A long acting liquid sulfonamide injectable composition comprising (a) water, (b) from 10 to 20% w/v based on the total volume of the solution of sulfadimethoxine, (c) from 50 to 65% w/v based on the total volume of the solution of 2-pyrrolidone and (d) from 1 to 5% w/v based on the total volume of the solution of polyvinylpyrrolidone having a m.w. of 5000—100,000, the concentration having a pH from 5 to 9.5.

4. A composition as claimed in any one of the preceding claims wherein an additional co-solvent selected from propylene glycol, a poly-ethylene glycol, benzyl alcohol, dimethylacet-amide, ethyl lactate and glycerol formal is present in an amount of from 0.5 to 30% w/v.

## Claims for the contracting state: AT

1. A process for preparing a long acting liquid sulfonamide injectable composition which comprises forming a composition comprising (a) water, (b) from 5 to 25% w/v based on the total volume of the solution of sulfadimethoxine and (c) 40 to 65% w/v based on the total volume of the solution of a co-solvent which is 2-pyrrolidone, the composition having a pH of from 5 to 9.5.

2. A process as claimed in claim 1, which comprises including in the composition from 1 to 5% w/v based on the total volume of the solution of polyvinylpyrrolidone having a m.w. of 5000—100,000.

3. A process for preparing a long acting liquid sulfonamide injectable composition which comprises forming a composition comprising (a) water, (b) from 10 to 20% w/v based on the total volume of the solution of sulfadimethoxine (c) from 50 to 65% w/v based on the total volume of the solution of 2-pyrrolidone and (d) from 1 to 5% w/v based on the total volume of the solution of polyvinylpyrrolidone having a m.w. of 5000—100,000, the composition having a pH from 5 to 9.5.

4. A process according to any one of the preceding claims wherein an additional co-solvent selected from propylene glycol, a polyethylene glycol, benzyl alcohol, dimethylacetamide, ethyl lactate and glycerol formal is included in a concentration of from 0.5 to 30% w/v.

5. A process as claimed in any one of the preceding claims, which comprises dissolving the sulfadimethoxine in aqueous 2-pyrrolidone, the aqueous 2-pyrrolidone containing the polyvinylpyrrolidone or additional co-solvent, if present, and, if necessary, adjusting the pH to from 5 to 9.5.

## Patentansprüche für die Vertragsstaaten: BE, CH, LI, DE, FR, GB, IT, LU, NL, SE

1. Lanwirksame injizierbare flüssige Sulfonamidzusammensetzung enthaltend (a) Wasser (b) 5 bis 25% Gewicht/Volumen, bezogen auf das Gesamtvolumen der Lösung, an Sulfadimethoxin und (c) 40 bis 65% Gewicht/Volumen, bezogen auf das Gesamtvolumen der Lösung, eines Co-Lösungsmittels, welches 2-Pyrrolidon ist, wobei die Zusammensetzung einen pH-Wert von 5 bis 9,5 aufweist.

2. Zusammensetzung gemäß Anspruch 1 welche zusätzlich 1 bis 5% Gewicht/Volumen, bezogen auf das Gesamtvolumen der Lösung, Polyvinylpyrrolidon mit einem Molekulargewicht von 5000 bis 100 000 enthält.

3. Langwirksame, injizierbare flüssige Sulfonamidzusammensetzung enthaltend (a) Wasser (b) 10 bis 20% Gewicht/Volumen bezogen auf das Gesamtvolumen der Lösung an Sulfandimethoxin (c) 50 bis 65% Gewicht/Volumen, bezogen auf das Gesamtvolumen der Lösung, an 2-Pyrrolidon und (d) 1 bis 5% Gewicht/Volumen bezogen auf das Ge-samtvolumen der Lösung, an Polyvinylpyrro-lidon mit einem Molekulargewicht von 5000 bis 100 000, wobei die Zusammensetzung einen pH-Wert von 5 bis 9,5 besitzt.

4. Zusammensetzung gemäß einem der vorgehenden Ansprüche worin ein zusätzliches Co-Lösungsmittel ausgewählt unter Propylenglycol, einem Polyäthylenglycol, Benzylalkohol, Dimethylacetamid, Äthyllactat und Glycerinformal in einer Menge von 0,5 bis 30% Gewicht/Volumen anwesend ist.

## Patentansprüche für den Vertragsstaat: AT

1. Verfahren zur Herstellung einer langwirk-enden flüssigen injizierbaren Sulfonamidzu-sammensetzung, welches das Bilden einer Zusammensetzung umfassend (a) Wasser, (b) 5 bis 25% Masse/Vol., bezogen auf das Gesamtvolumen der Lösung, Sulfadimethoxin und (c) 40 bis 65% Masse/Vol., bezogen auf das Ge-samtvolumen der Lösung, eines Colösungsmit-tels, das 2-Pyrrolidon ist, umfaßt, wobei die Zusammensetzung einen pH von 5 bis 9,5 aufweist.

2. Verfahren wie in Anspruch 1 beansprucht, welches das Einverleiben von 1 bis 5%-Masse/Vol., bezogen auf das Gesamtvolumen der Lösung, Polyvinylpyrrolidon mit einer Molmasse von 5000 bis 100 000 in die Zusammensetzung umfaßt.

3. Verfahren zur Herstellung einer langwirk-enden flüssigen injizierbaren Sulfonamidzu-sammensetzung, welches das Bilden einer Zusammensetzung umfassend (a) Wasser, (b) 10 bis 20%-Masse, bezogen auf das Gesamtvolumen der Lösung, Sulfadimethoxin, (c) 50 bis 65%-Masse/Vol., bezogen auf das Gesamtvolumen der Lösung, 2-Pyrrolidon und (d) 1 bis 5%-Masse/Vol., bezogen auf das Gesamtvolumen der Lösung, Polyvinylpyrrolidon mit einer Molmasse von 5000 bis 100 000 umfaßt, wobei die Zusammensetzung einen pH von 5 bis 9,5 aufweist.

4. Verfahren gemäß einem der vorhergehenden Ansprüche, wobei ein zusätzliches Colösungsmittel ausgewählt unter Propylenglykol, einem Polyäthylenglykol, Benzylalkohol, Dimethylacetamid, Äthyllactat und Glycerinformal in einer Konzentration von 0,5 bis 30% Masse/Vol. einverleibt wird.

5. Verfahren wie in einem der vorhergehenden Ansprüche beansprucht, welches das Lösen des Sulfadimethoxins in wässerigem 2-Pyrrolidon, wobei das wässerige 2-Pyrrolidon das Polyvinylpyrrolidon oder zusätzliches Colösungsmittel, wenn vorhanden, enthält, und, wenn notwendig , Einstellen des pH auf 5 bis 9,5 umfaßt.

### Revendications pour les etats: BE, CH, LI, DE, FR, GB, IT, LU, NL, SE

1. Composition liquide injectable contenant un sulfamide, à longue durée d'action, comprenant (a) de l'eau, (b) 5 à 25% en poids/volume sur la base du volume total de la solution, de sulfadiméthoxine et (c) 40 à 65% en poids/volume, sur la base du poids total de la solution, d'un cosolvant qui est la 2-pyrrolidone, la composition ayant un pH de 5 à 9,5.

2. Composition suivant la revendication 1, qui comprend en outre 1 à 5% en poids/volume, sur la base du volume total de la solution, de polyvinylpyrrolidone ayant un poids moléculaire de 5000 à 100 000.

3. Composition liquide injectable contenant un sulfamide, à longue durée d'action, comprenant (a) de l'eau, (b) 10 à 20% en poids/volume, sur la base du volume total de la solution, de sulfadiméthoxine, (c) 50 à 65% en poids/volume, sur la base du volume total de la solution, de 2-pyrrolidone et (d) 1 à 5% en poids/volume, sur la base du volume total de la solution, de polyvinylpyrrolidone ayant un poids moléculaire de 5000 à 100 000, la composition ayant un pH de 5 à 9,5.

4. Composition suivant l'une quelconque des revendications précédentes, dans laquelle un autre cosolvant choisi entre le propylène-glycol, un polyéthylène-glycol, l'alcool benzylique, le diméthacétamide, le lactate d'éthyle et le formal de glycérol est présent en une quantité de 0,5 à 30% en poids/volume.

### Revendications pour l'etat: AT

1. Procédé de préparation d'une composition liquide injectable contenant un sulfamide, à longue durée d'action, qui consiste à former une composition comprenant (a) de l'eau, (b) 5 à 25% en poids/volume, sur la base du volume total de la solution, de sulfadiméthoxine et (c) 40 à 65% en poids/volume, sur la base du volume total de la solution, d'un cosolvant qui est la 2-pyrrolidone, la composition ayant un pH de 5 à 9,5.

2. Procédé suivant la revendication 1, qui consiste à inclure dans la composition 1 à 5% en poids/volume, sur la base du volume total de la solution, de polyvinylpyrrolidone ayant un poids moléculaire de 5000 à 100 000.

3. Procédé de préparation d'une composition liquide injectable contenant un sulfamide, à longue durée d'action, qui consiste à former une composition comprenant (a) de l'eau, (b) 10 à 20% en poids/volume, sur la base du volume total de la solution, de sulfadiméthoxine, (c) 50 à 65% en poids/volume, sur la base du volume total de la solution, de 2-pyrrolidone et (d) 1 à 5% en poids/volume, sur la base du volume total de la solution, de polyvinylpyrrolidone ayant un poids moléculaire de 5000 à 100 000, la composition ayant un pH de 5 à 9,5.

4. Procédé suivant l'une quelconque des revendications précédentes, dans lequel un autre cosolvant choisi entre le propylène-glycol un polyéthylène-glycol, l'alcool benzylique, le diméthylacétamide, le lactate d'éthyle et le formal du glycérol est inclus à une concentration de 0,5 à 30% en poids/volume.

5. Procédé suivant l'une quelconque des revendications précédentes, qui consiste à dissoudre la sulfadiméthoxine dans de la 2-pyrrolidone aqueuse, la 2-pyrrolidone aqueuse contenant la polyvinylpyrrolidone ou un autre cosolvant éventuellement présent et, le cas échéant, à ajuster le pH à une valeur de 5 à 9,5.